# EUROPEAN PATENT APPLICATION

(11) **EP 2 843 461 A1**
(43) Date of publication of application: **04.03.2015**
(21) Application number: 14182584.4
(22) Date of filing: 28.08.2014
(51) Int. Cl.: G02C 7/04

(54) **Methods and apparatus to form ophthalmic devices incorporating fluorescence detectors**

(30) Priority: 28.08.2013 US 201314011902
(71) Applicant: Johnson & Johnson Vision Care, Inc., Jacksonville, FL 32256 (US)
(72) Inventor: Pugh, Randall B., Jacksonville, FL Florida 32259 (US); Flitsch, Frederick A., New Windsor, NY New York 12553 (US)
(74) Representative: Carpmaels & Ransford LLP

(57) **Abstract**

Ophthalmic devices with media inserts that have fluorescence based analysis elements upon or within them may be utilized to analyze and monitor fluid for certain components. Methods and devices for active ophthalmic devices based on fluorescence based analysis elements may also be formed. More specifically, the fluorescence based analysis elements may be useful for analyzing an analyte such as glucose in a fluid sample.

## Description

### BACKGROUND OF THE INVENTION

### 1. Field of the Invention

The present invention relates to ophthalmic devices that have fluorescence detectors upon or within them. The ophthalmic devices may be used for analyzing an analyte in fluids in an ophthalmic environment.

### 2. Discussion of the Related Art

Traditionally, an ophthalmic device, such as a contact lens, an intraocular lens, or a punctal plug, included a biocompatible device with a corrective, cosmetic, or therapeutic quality. A contact lens, for example, may provide one or more of vision correcting functionality, cosmetic enhancement, and/or therapeutic effects. Each function is provided by a physical characteristic of the lens. A design incorporating a refractive quality into a lens may provide a vision corrective function. Pigmentation incorporated into the lens may provide a cosmetic enhancement. An active agent incorporated into a lens may provide a therapeutic functionality. Such physical characteristics are accomplished without the lens entering into an energized state. An ophthalmic device has traditionally been a passive device.

Novel ophthalmic devices based on energized ophthalmic inserts have recently been described. These devices may use the energization function to power active optical components.

In a related field, recently developed glucose detecting devices that are implanted into ophthalmic tissue have been described. The devices and future alternatives may utilize Förster resonance energy transfer (FRET) probes to produce a resonance signal that is sensitive to the concentration of glucose near the probe. Exemplary devices may include implants placed into relatively transparent tissue in the eye to probe for glucose concentration in Interstitial Fluid (ISF) in the ophthalmic environment.

Some of these devices are probed by hand held fluorescence detectors. Contact lenses, which incorporate fluorescence detector arrangements, may provide convenient and improved means for detecting glucose concentrations. Therefore, it may be useful to design ophthalmic devices to which may incorporate energized fluorescence detectors to analyze glucose levels.

### SUMMARY OF THE INVENTION

The ophthalmic devices incorporating fluorescence detectors in accordance with the present invention overcome the disadvantages associated with the prior art as briefly described above. Accordingly, the present invention includes an encapsulated media insert that comprises a fluorescence analyzer component.

In some exemplary embodiments, an ophthalmic lens media insert device may be formed which comprises an annular media insert comprising a sealed component cavity within the annular media insert. The annular media insert may be formed from a front annular insert piece and a rear annular insert piece which are joined together. The rear annular insert piece may equivalently be referred to as a back annular insert piece. In some exemplary embodiments, the front annular insert piece may be sealed to the rear annular insert piece. The sealing may occur in various manners and locations and in some exemplary embodiments both an inner annular seal location and an outer annular seal location may be formed and sealed. When an annular media insert is sealed in such a manner, the region between the various seals and the annular front insert piece and the annular back insert piece may be a cavity in which various components may be located.

In some exemplary embodiments, an energy source may be a component that may be located in the sealed component cavity within the annular media insert. In other exemplary embodiments, an energy source may be located externally or in part externally to the sealed component cavity, but have the ability to make electrical connection to components within the sealed cavity.

In some exemplary embodiments the ophthalmic lens media insert device may also comprise interconnects that may be at least partially located within the sealed component cavity.

In some exemplary embodiments, an electronic circuit may be located within the sealed component cavity.

In some exemplary embodiments, a tab of material may protrude from the annular insert piece from either the front annular insert piece, the back or rear annular insert piece or as a connected feature to either of these. In some exemplary embodiments, the tab may support a fluorescence analysis component along at least portions of the surface of the annular insert piece. The surface portions may be located externally to the sealed component cavity in some exemplary embodiments or it may be located within a sealed component cavity that may be located within the tab region or may be connected to the tab region. The fluorescence analysis component may connect to a portion of the interconnects and may be in electrical communication with electronic circuits within the annular media insert device.

In some exemplary embodiments, a tab region may be formed on the front annular surface and may also be formed on the rear or back annular insert piece. The fluorescence analysis component may be supported upon the tab region of the rear annular insert piece. In other exemplary embodiments, the fluorescence analysis component may be supported upon the tab region of the front annular insert piece. In still other exemplary embodiments, the fluorescence analysis component may be located in proximity to either or both of the front annular insert piece and the rear annular insert piece, but may not be directly affixed to either piece. In some exemplary embodiments, there may a tab region formed on only one of the front annular insert piece or the rear annular insert piece.

In some exemplary embodiments, an ophthalmic contact lens device may be formed utilizing the annular media insert device. In some exemplary embodiments, a media insert device consistent with the previous descriptions may be encapsulated or shrouded within a skirt of material that may be consistent with interaction with human tissue in an ophthalmic environment. In some exemplary embodiments, the skirt may comprise a hydrogel material.

A fluorescence probe may be located proximate the tissue of a human eye environment and may therefore have a fixed location and orientation in that environment. It may be important for an ophthalmic contact lens device to maintain an orientation so that a fluorescence analysis component may correctly interact with a fluorescence probe. The skirt of material that enshrouds or encapsulates the media insert may include structural features that may be useful in orienting the contact lens. These devices which may be referred to as stabilization features or zones may keep the ophthalmic device in a correct orientation through their interaction with the ocular environment including, for example, the eyelids of a user.

The various exemplary fluorescence detection embodiments may include electronic components to control functions, perform analysis functions, retain data and similar such functions as well as communicate data to electronic components, receivers or transceivers located outside the fluorescence detection device. In some exemplary embodiments, some or all of the electronic components may be in a stacked integrated component form.

In some exemplary embodiments, an ocular fluid analysis system may comprise an ophthalmic device. The ophthalmic device may comprise energization elements or batteries where the elements are a portion of the ophthalmic device. The ophthalmic device may in some exemplary embodiments be suitable to be worn by a user while in contact with ocular fluid of the user's eye. A fluorescence analysis system may be in electrical communication with the energy source or sources. The fluorescence analysis system may be configured operatively to measure a fluorescence signal in the ocular environment.

In some exemplary embodiments, a fluorescence analysis probe may be located within ocular tissue of the user in such a manner that the portion of the ophthalmic device comprising the fluorescence analysis system may interact therewith. The fluorescence analysis probe may interact with at least a first compound that is located in the interstitial tissue spaces that surround the fluorescence analysis probe. The fluorescence analysis probe may interact with the first compound in such a manner that the interaction causes the probe to emit a characteristic signal based at least in part upon the concentration of the first compound that it interacts with. The emission of the characteristic signal may be predicated, triggered and/or supported by interaction with an excitation signal that it may receive from the fluorescence analytical system. In some exemplary embodiments, a light-based excitation signal may be emanated by the fluorescence analytical system under control of electronic circuitry within the device. The light-based excitation signal may be absorbed within the fluorescence analysis probe and the probe may subsequently emit the characteristic signal which may be a fluorescence signal. The electronic circuitry may comprise a processor that may form a portion or part of the ophthalmic device. The processor may be capable of executing a program, and the processor may be capable of storing values or data related to the fluorescence signal observed by the fluorescence analysis system. In some exemplary embodiments, the processor may cause or be configured to emanate or transmit or otherwise output a signal. In some exemplary embodiments, the transmission may be caused or initiated based on receiving a transmission of a signal that is sent from external to the device. The output signal may encode in various fashions the stored values into a transmittable signal.

In still some other exemplary embodiments, the processor, which may be capable of executing a program, may evaluate a detected fluorescence signal against a preprogrammed threshold value. In yet other exemplary embodiments, the threshold value may be received from a transmission from an external source. The threshold value may relate to one or more ocular fluid properties that may be indicated by the fluorescence signal characteristics. In some exemplary embodiments, when the evaluated detected fluorescence signal is outside the limit relating to the threshold value the processor or electronic circuitry may cause the outputting of a signal. The signal may be received by an external transceiver or receiver and cause an alarm or other action to be precipitated external to the ophthalmic device in some exemplary embodiments.

The ophthalmic device that incorporates fluorescence detectors may be utilized in various manners. In some exemplary embodiments, an ophthalmic device comprising a fluorescence analytical system may be provided to a user. The user may, for example, wear the ophthalmic device as a contact lens device. In some exemplary embodiments, there may be a reception of a data value external to the ophthalmic device of a transmission that originates from the ophthalmic contact lens. In other words, the ophthalmic device may be configured for one or two way communication with external devices.

### DESCRIPTION OF THE DRAWINGS

The foregoing and other features and advantages of the invention will be apparent from the following, more particular description of preferred embodiments of the invention, as illustrated in the accompanying drawings.
Figs. 1A - 1B illustrate an exemplary embodiment of a media insert for an energized ophthalmic device and an exemplary embodiment of an energized ophthalmic device.
Figs. 2A - 2B illustrate an exemplary annular shaped insert and a cross sectional representation at an indicated location.
Fig. 3 illustrates an exemplary embodiment of a media insert with a fluorescence sensor located in a peripheral location in electrical communication with an energized electronic circuit element.
Figs. 4A - 4B illustrate an exemplary embodiment of an ophthalmic contact lens with a media insert that comprises a fluorescence sensor. Other features of an exemplary contact lens are also depicted.
Figs 5A- 5B illustrate an exemplary ophthalmic environment with an exemplary fluorescence probe for an analyte. A corresponding overlay of an ophthalmic lens in the ophthalmic environment is also depicted.
Fig. 6 illustrates an exemplary ophthalmic lens in an ophthalmic environment comprising a fluorescence probe for an analyte transferring information wirelessly to a receiving unit.
Fig. 7 demonstrates an exemplary stacked die implementation of fluorescence detection elements incorporated within ophthalmic devices.
Fig. 8 demonstrates a processor that may be used to implement exemplary embodiments of the present invention.

### DETAILED DESCRIPTION OF THE PREFERRED EMBODIMENTS

The present invention relates to an ophthalmic device having sensing elements capable of stimulating a fluorescence probe and sensing the fluorescent emissions from the probe. In the following sections detailed descriptions of exemplary embodiments of the invention are given. The description of both preferred and alternative embodiments are exemplary embodiments only, and it is understood that to those skilled in the art that variations, modifications and alterations may be apparent. It is therefore to be understood that the exemplary embodiments do not limit the scope of the underlying invention.

### Glossary

In the description and claims directed to the present invention, various terms may be used for which the following definitions will apply:
"Electro-wetting on Dielectric" or "EWOD": as used herein refers to a class of devices or a class of portions of devices where a combination of immiscible fluids or liquids, a surface region with defined surface free energy and an electro-potential field are present. Typically, the electro-potential field will alter the surface free energy of the surface region, which may alter the interaction of the immiscible fluids with the surface region.
"Energized": as used herein refers to the state of being able to supply electrical current to or to have electrical energy stored within.
"Energy": as used herein refers to the capacity of a physical system to do work. Many uses within the present invention may relate to the capacity being able to perform electrical actions in doing work.
"Energy Source": as used herein refers to a device or layer that is capable of supplying energy or placing a logical or electrical device in an energized state.
"Energy Harvester": as used herein refers to a device capable of extracting energy from the environment and converting it to electrical energy.
"Fluorescent probe": as used herein refers to a chemical probe that interacts with its environment to alter a fluorescence property that the probe manifests.
"Fluorescence based analysis": as used herein refers to the use of a fluorescence property in performing a chemical based analysis.
"Fluorophore": as used herein refers to a fluorescent chemical compound that may be bound or combined with other chemical compounds that can re-emit light upon light excitation.
"Functionalized": as used herein refers to making a layer or device able to perform a function including for example, energization, activation, or control.
"Leakage": as used herein refers to unwanted loss of energy.
"Lens" or "Ophthalmic Device": as used herein refers to any device that resides in or on the eye. These devices may provide optical correction, may be cosmetic, or may provide functionality unrelated to the eye. For example, the term lens may refer to a contact lens, intraocular lens, overlay lens, ocular insert, optical insert, or other similar device through which vision is corrected or modified, or through which eye physiology is cosmetically enhanced (e.g. iris color) without impeding vision. Alternatively, the lens may provide non-optic functions, for example, monitoring glucose or administrating medicine. In some embodiments, the preferred lenses of the present invention are soft contact lenses are made from silicone elastomers or hydrogels, which include, for example, silicone hydrogels, and fluorohydrogels.
"Lens-forming mixture" or "Reactive Mixture" or "Reactive Monomer Mixture" (RMM): as used herein refers to a monomer or prepolymer material that may be cured and crosslinked or crosslinked to form an ophthalmic lens. Various embodiments may include lens-forming mixtures with one or more additives, for example, UV blockers, tints, photoinitiators or catalysts, and other additives one might desire in an ophthalmic lenses such as, contact lenses or intraocular lenses.
"Lens-forming Surface": as used herein refers to a surface that is used to mold a lens. In some exemplary embodiments, any such surface may have an optical quality surface finish, which indicates that it is sufficiently smooth and formed so that a lens surface fashioned by the polymerization of a lens forming material in contact with the molding surface is optically acceptable. Further, in some embodiments, the lens-forming surface may have a geometry that is necessary to impart to the lens surface the desired optical characteristics, including spherical, aspherical and cylinder power, wave front aberration correction, corneal topography correction and the like as well as any combinations thereof.
"Lithium Ion Cell": as used herein refers to an electrochemical cell where Lithium ions move through the cell to generate electrical energy. This electrochemical cell, typically called a battery, may be reenergized or recharged in its typical forms.
"Media Insert": as used herein refers to an encapsulated insert that will be included in an energized ophthalmic device. The energization elements and circuitry may be incorporated in the media insert. The media insert defines the primary purpose of the energized ophthalmic device. For example, in embodiments where the energized ophthalmic device allows the user to adjust the optic power, the media insert may include energization elements that control a liquid meniscus portion in the optical zone. Alternatively, a media insert may be annular so that the optical zone is void of material. In such embodiments, the energized function of the lens may not be optic quality, but may be, for example, monitoring glucose or administering medicine.
"Mold": as used herein refers to a rigid or semi-rigid object that may be used to form lenses from uncured formulations. Some preferred molds include two mold parts forming a front curve mold part and a back curve mold part.
"Operating Mode": as used herein refers to a high current draw state where the current over a circuit allows the device to perform its primary energized function.
"Optical Zone": as used herein refers to an area of an ophthalmic lens through which a wearer of the ophthalmic lens sees.
"Power": as used herein refers to work done or energy transferred per unit of time.
"Rechargeable" or "Re-energizable": as used herein refers to a capability of being restored to a state with higher capacity to do work. Many uses within the present invention may relate to the capability of being restored with the ability to flow electrical current at a certain rate and for a certain, reestablished period.
"Reenergize" or "Recharge": as used herein refers to restoring to a state with higher capacity to do work. Many uses within this invention may relate to restoring a device to the capability to flow electrical current at a certain rate and for a certain, reestablished period.
"Released from a Mold": as used herein refers to a lens that is either completely separated from the mold, or is only loosely attached so that it may be removed with mild agitation or pushed off with a swab.
"Stacked": as used herein means to place at least two component layers in proximity to each other such that at least a portion of one surface of one of the layers contacts a first surface of a second layer. In some exemplary embodiments, a film, whether for adhesion or other functions may reside between the two layers that are in contact with each other through said film.
"Stacked Integrated Component Devices" or "SIC Devices": as used herein refers to the products of packaging technologies that assemble thin layers of substrates that may include electrical and electromechanical devices into operative-integrated devices by means of stacking at least a portion of each layer upon each other. The layers may comprise component devices of various types, materials, shapes, and sizes. Furthermore, the layers may be made of various device production technologies to fit and assume various contours.
"Storage Mode": as used herein refers to a state of a system comprising electronic components where a power source is supplying or is required to supply a minimal designed load current. This term is not interchangeable with standby mode.
"Substrate Insert": as used herein refers to a formable or rigid substrate capable of supporting an energy source within an ophthalmic lens. In some exemplary embodiments, the substrate insert also supports one or more components.

### Energized Ophthalmic Device

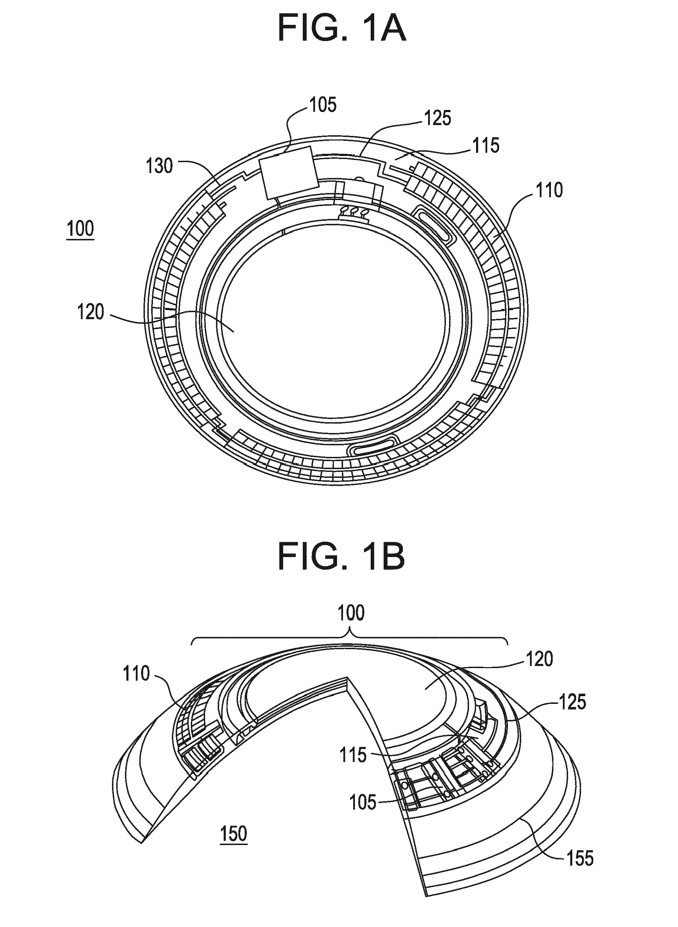

Referring to Fig. 1A, an exemplary embodiment of a media insert 100 for an energized ophthalmic device and a corresponding energized ophthalmic device 150 (Fig. 1B) are illustrated. The media insert 100 may comprise an optical zone 120 that may or may not be functional to provide vision correction. Where the energized function of the ophthalmic device is unrelated to vision, the optical zone 120 of the media insert may be void of material. In some exemplary embodiments, the media insert may include a portion not in the optical zone 120 comprising a substrate 115 incorporated with energization elements 110 (power source) and electronic components 105 (load).

In some exemplary embodiments, a power source 110, for example, a battery, and a load 105, for example, a semiconductor die, may be attached to the substrate 115. Conductive traces 125 and 130 may electrically interconnect the electronic components 105 and the energization elements 110. The media insert may be fully encapsulated to protect and contain the energization elements 110, traces 125, and electronic components 105. In some exemplary embodiments, the encapsulating material may be semi-permeable, for example, to prevent specific substances, such as water, from entering the media insert and to allow specific substances, such as ambient gasses or the byproducts of reactions within energization elements, to penetrate or escape from the media insert.

In some exemplary embodiments, as depicted in Fig. 1B, the media insert 100 may be included in an ophthalmic device 150, which may comprise a polymeric biocompatible material. The ophthalmic device 150 may include a rigid center, soft skirt design wherein the central rigid optical element comprises the media insert 100. In some specific embodiments, the media insert 100 may be in direct contact with the atmosphere and the corneal surface on respective anterior and posterior surfaces, or alternatively, the media insert 100 may be encapsulated in the ophthalmic device 150. The periphery 155 of the ophthalmic device or lens 150 may be a soft skirt material, including, for example, a hydrogel material. The infrastructure of the media insert 100 and the ophthalmic device 150 may provide an environment for numerous embodiments involving fluid sample processing with fluorescence based analysis elements.

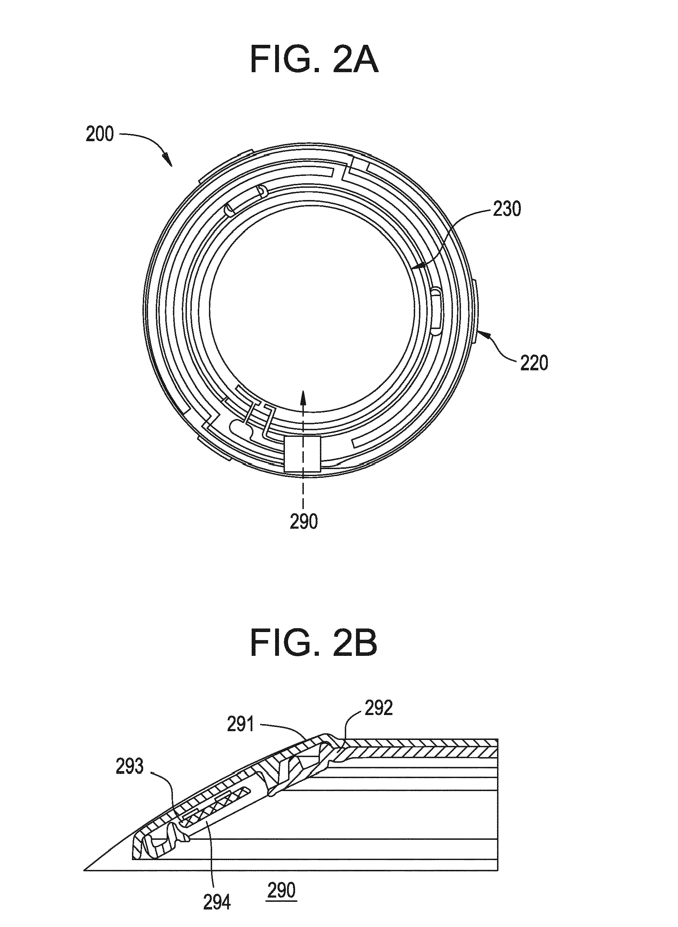

Referring to Fig. 2A - 2B, a depiction of an exemplary multi-piece insert 200 in annular form is illustrated in both plan view, Fig. 2A, and cross section view, Fig. 2B. The insert 200 is an annular insert with a ring of material around a central optical zone that is devoid of material. The annular insert 200 comprises an exterior extent as shown by item 220 and an internal annulus edge at item 230. Included in the insert 200 may be found energization elements, interconnect features of various types and electronic circuit elements.

A dashed line at 290 represents a cross sectional direction. In the detail of Fig. 2B, shown as 290, is a cross section along the direction of the dashed line. The cross section reveals that the insert 200 may be a combination of a front insert piece 291 and a rear insert piece 292. Various means of joining and sealing these two pieces along the various surfaces of the annulus may be defined, and an exemplary sealing design is depicted. Also shown in an encapsulated location may be an integrated circuit element 293 connected to interconnection elements. In some exemplary embodiments, the rear insert piece 292 may have a gap in the region of an integrated circuit 293. In these exemplary embodiments, integrated circuit 293 may include a sensor that may function in an improved fashion if it may sense emanations without the perturbing aspects of an insert piece.

### Fluorescence Based Probe Elements for Analyte Analysis

Various types of analytes may be detected and analyzed using fluorescence based analysis techniques. A subset of these techniques may involve the direct fluorescence emission from the analyte itself. A more generic set of techniques relate to fluorescence probes that have constituents that bind to analyte molecules and in so binding alter a fluorescence signature. For example, in Förster Resonance Energy Transfer (FRET), probes are configured with a combination of two fluorophores that may be chemically attached to interacting proteins. The distance of the fluorophores from each other can affect the efficiency of a fluorescence signal emanating therefrom One of the fluorophores may absorb an excitation irradiation signal and can resonantly transfer the excitation to electronic states in the other fluorophore. The binding of analytes to the attached interacting proteins may disturb the geometry and cause a change in the fluorescent emission from the pair of fluorophores. Binding sites may be genetically programmed into the interacting proteins, and for example, a binding site, which is sensitive to glucose, may be programmed. In some cases, the resulting site may be less sensitive or non-sensitive to other constituents in interstitial fluids of a desired sample.

The binding of an analyte to the FRET probes may yield a fluorescence signal that is sensitive to glucose concentrations. In some exemplary embodiments, the FRET based probes may be sensitive to as little as a 10 uM concentration of glucose and may be sensitive to concentrations up to hundreds of micro molar. Various FRET probes may be genetically designed and formed. The resulting probes may be configured into structures that may assist analysis of interstitial fluids of a subject. In some exemplary embodiments, the probes may be placed within a matrix of material that is permeable to the interstitial fluids and their components, for example, the FRET probes may be assembled into hydrogel structures.

In some exemplary embodiments, these hydrogel probes may be included into the hydrogel based processing of ophthalmic contact lenses in such a manner that they may reside in a hydrogel encapsulation that is immersed in tear fluid when worn upon the eye. In other exemplary embodiments, the probe may be inserted in the ocular tissues just above the sclera. A hydrogel matrix comprising fluorescence emitting analyte sensitive probes may be placed in various locations that are in contact with bodily fluids containing an analyte.

In the examples provided, the fluorescence probes may be in contact with interstitial fluid of the ocular region near the sclera. In these cases, where the probes are invasively embedded, a sensing device may provide a radiation signal incident upon the fluorescence probe from a location external to the eye such as from an ophthalmic lens or a hand held device held in proximity to the eye.

In other exemplary embodiments, the probe may be embedded within an ophthalmic lens in proximity to a fluorescence-sensing device that is also embedded within the ophthalmic lens. In some exemplary embodiments, a hydrogel skirt may encapsulate both an ophthalmic insert with a fluorescence detector as well as a FRET based analyte probe.

### Ophthalmic Insert Devices and Ophthalmic Devices with Fluorescence Detectors

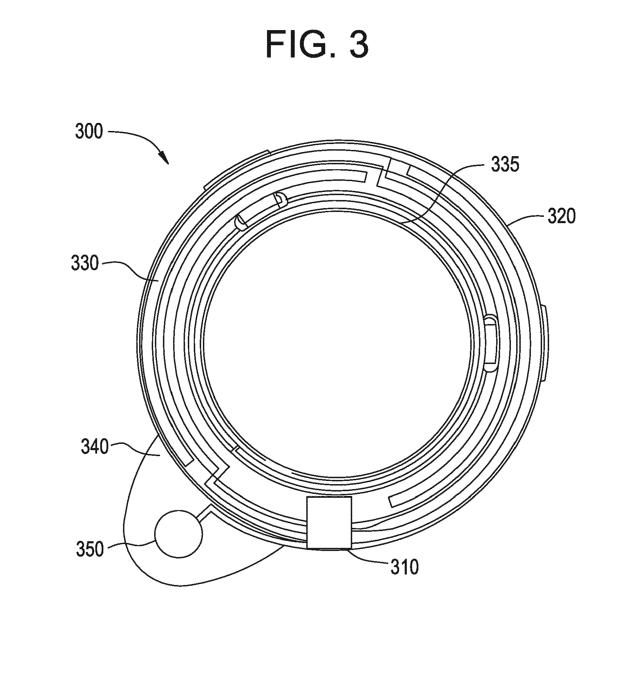

Referring to Fig. 3, an ophthalmic insert is demonstrated including components that may form an exemplary fluorescence based analytical system. The demonstrated ophthalmic insert is shown in an exemplary annular form having an internal border of 335 and an external border of 320. In addition to energization elements 330, control circuitry 310, and interconnect features 360 there may be a fluorescence analytical system 350, which in certain exemplary embodiments may be positioned on a flap 340. The flap 340 may be connected to the insert 300 or be an integral, monolithic extension thereof. The flap 340 may properly position the fluorescence analytical system 350 when an ophthalmic device comprising a fluorescence is detector is worn. The flap 340 may allow the analytical system 350 to overlap with portions of the user's eye away from the optic zone. The fluorescence based analytical system 350 may be capable of determining an analyte, in terms of its presence or its concentration, in a fluid sample. As a non-limiting example, the fluorophores may include Fluorescein, Tetramethylrhodamine, or other derivatives of Rhodamine and Fluorescein. It may be obvious to those skilled in the art that any fluorescence emitting analyte probe, which may include fluorophore combinations for FRET or other fluorescence-based analysis may be consistent with the art herein.

For a fluorescence analysis, a probe may be irradiated with an excitation light source. This light source may be located within the body of the analytical system 350. In some exemplary embodiments, the light source may comprise a solid-state device or devices such as a light emitting diode. In an alternative exemplary embodiment, an InGaN based blue laser diode may irradiate at a frequency corresponding to a wavelength of 442 nm for example. Nanoscopic light sources as individual or array sources may be formed from metallic cavities with shaped emission features such as bowties or crosses. In other exemplary embodiments, light emitting diodes may emit a range of frequencies at corresponding wavelengths that approximate 440 nm, for example. As well, the emission sources may be supplemented with a band pass filtering device in some embodiments.

Other optical elements may be used to diffuse the light source from the solid-state device as it leaves the insert device. These elements may be molded into the ophthalmic insert body itself. In other exemplary embodiments, elements such as fiber optic filaments may be attached to the insert device to function as a diffuse emitter. There may be numerous means to provide irradiation to a fluorescence probe from an ophthalmic insert device 300 of the type demonstrated in Fig. 3.

A fluorescence signal may also be detected within the fluorescence based analytical system 350. A solid-state detector element may be configured to detect light in a band around 525 nm as an example. The solid-state element may be coated in such a manner to pass only a band of frequencies that is not present in the light sources that have been described. In other exemplary embodiments, the light sources may have a duty cycle and a detector element's signal may only be recorded during periods when the light source is in an off state. When the duty cycle is used, detectors with wide band detection ability may be advantageous.

An electronic control bus of interconnects shown schematically as item 360 may provide the signals to the light source or sources and return signals from the detectors. The powered electronic component, item 310 may provide the signals and power aspects. The exemplary embodiment of Fig. 3, illustrates a battery power source 330 to the electronic circuitry 310. In other exemplary embodiments, energization may also be provided to the electronic circuitry by the coupling of energy through wireless manners such as radio frequency transfer or photoelectric transfer.

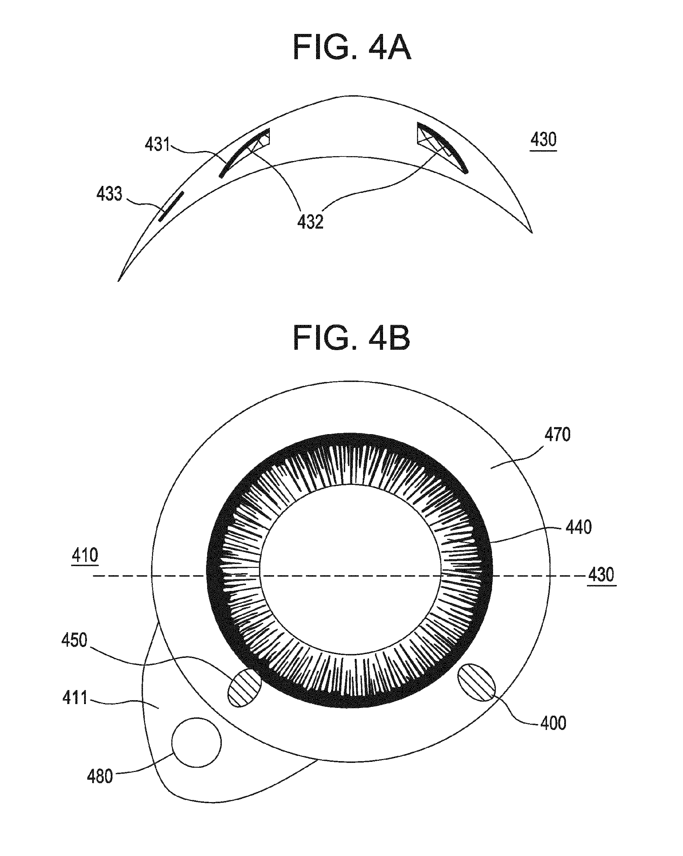

Referring to Figs.4A - 4B, an ophthalmic lens in the form of a contact lens with a protruding tab 411 is illustrated which has incorporated a fluorescence detector. In Fig. 4B, item 410 represents a top view of an ophthalmic lens. It includes an ophthalmic lens skirt 470. In some exemplary embodiments, the skirt 470 may be formed of the various hydrogel compounds consistent with the formation of contact lenses. In other exemplary embodiments, certain hydrogel compounds may be preferred for their properties relating to the various fluorescence probes that have been discussed.

A cross section is demonstrated by the dashed line 430. At Fig. 4A, a cross sectional depiction along dashed line 430 may be found. The insert device 300 described in Fig. 3 may be located within the ophthalmic lens and have the annular cross sectional representation 432 as depicted at 430. The lens is depicted with a printed iris pattern 440 that may be demonstrated on the annular pieces as item 431. Beneath the printed pattern may be insert components as described with reference to Fig. 3. The fluorescence detection element may be located at 433. The ophthalmic lens 410 may also have other features important to the function of a lens with a fluorescence detector. At 450 and 460, stabilization features may be included in the body of the formed ophthalmic device. These features may allow the lens 400 to be oriented in a preferred configuration when worn by a user. In exemplary embodiments where a fluorescence based analyte probe is embedded in a user's eye, the stabilization features 450 and 460 may be useful to aid in a good overlap between the embedded analyte probe and the fluorescence analysis element 480. In other exemplary embodiments where the analyte probe may be included in the hydrogel body 470 of the lens, the stabilization features 450 and 460 may be useful in orienting the analysis element and probe into a preferred region of the eye with tear fluid.

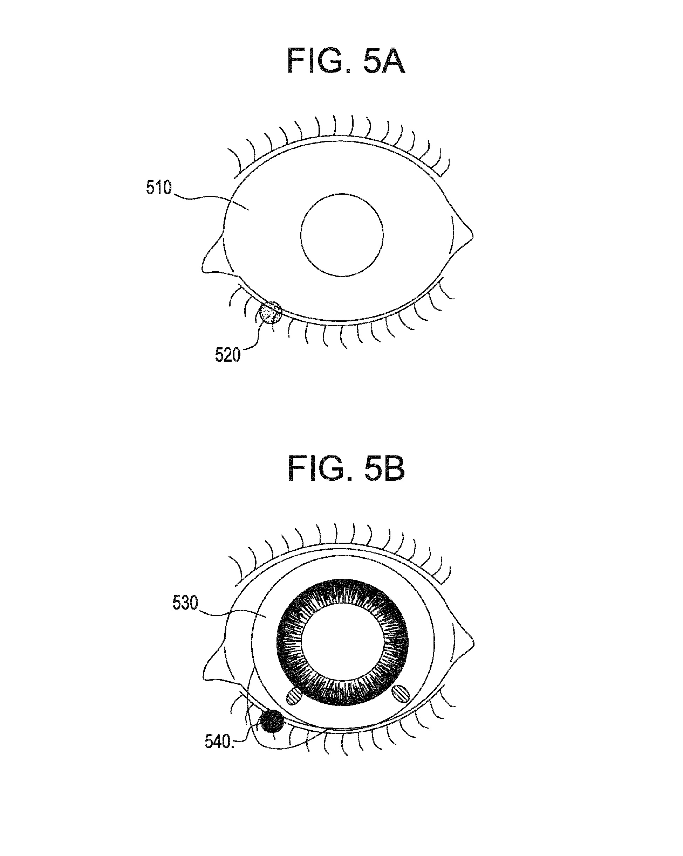

Referring to Figs. 5A and 5B, the use of an ophthalmic device incorporating fluorescence detectors in a user's eye 510 is illustrated in concert with a sub-tissue embedded analyte probe 520. As depicted, when the ophthalmic device 530 is placed upon a users eye and assumes an orientation that may be guided by features on the ophthalmic device it may locate a fluorescence based analysis element 540 in an overlap with the analyte probe 520. It may be useful to scale the size of the analysis element 540 to be larger than that of the probe 520 to allow for some flexibility to alignment of the two features.

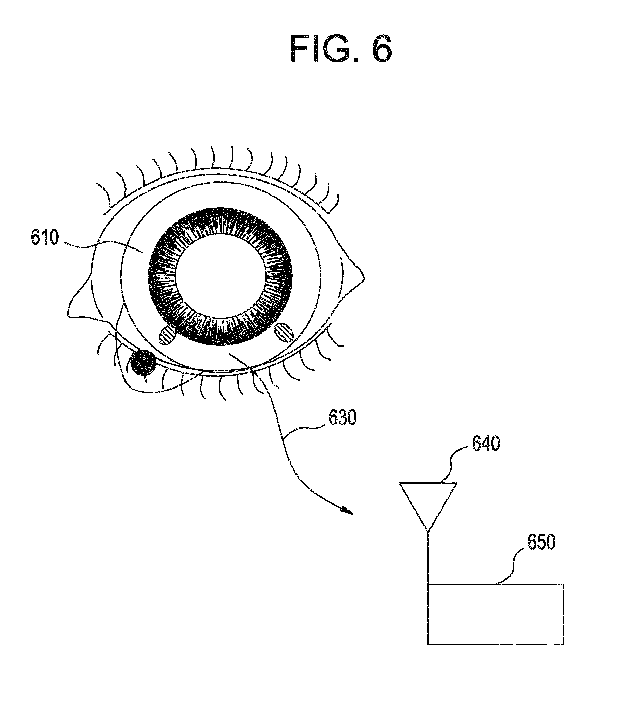

Referring to Fig. 6, the transfer of information from an ophthalmic device incorporating fluorescence detectors to an external data reception device is depicted. The ophthalmic lens 610 may be worn upon a user's eye for a period long enough to allow the embedded analysis element to equilibrate with its surroundings and to begin performing analysis for an analyte. Either as the data is collected or in other exemplary embodiments after the data has been collected, a data transfer protocol may be initiated. A wireless signal, represented by arrow 630, may be emitted from the ophthalmic lens 610 to communicate with an external reception device 650. A receiving element 640 may include an antenna for radiofrequency transmissions or other transducers to transform light-based signals, sound based signals or other wireless forms of communication into received information at the reception device 650.

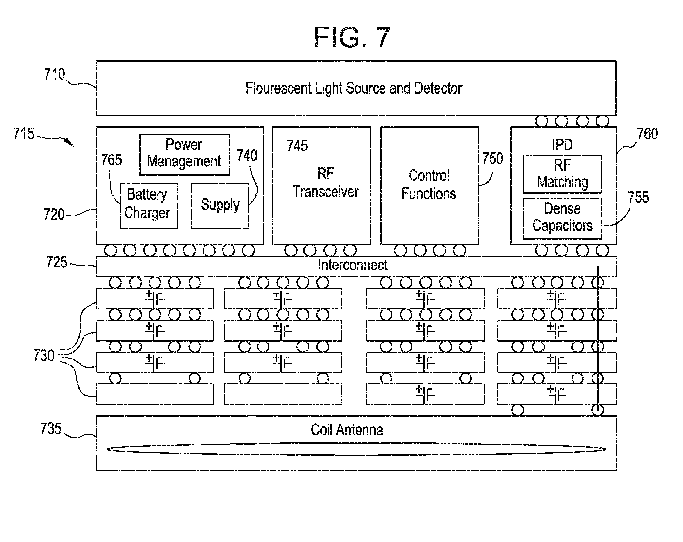

In some exemplary embodiments, the powered operation of an ophthalmic lens incorporating fluorescence detectors may function well with monolithic components included in the ophthalmic insert device. In other exemplary embodiments, such as that depicted in Fig. 7, a stacked integrated component may be useful to perform the various functions including power management 715, communications 745, control functions 750 and the transmission of light and reception of fluorescence signal 710.

In exemplary embodiments of this type, as depicted in Fig. 7, the media insert may include numerous layers of different types that are encapsulated into forms consistent with the ophthalmic environment that they will occupy. In some exemplary embodiments, these inserts with stacked integrated component layers may assume the entire annular shape of the insert. Alternatively, in some exemplary embodiments, the media insert may be an annulus whereas the stacked integrated component may occupy just a portion of the volume within the entire shape.

Continuing with the example of Fig. 7, a stacked integrated component media insert may assume numerous functional aspects. As shown in Fig. 7, there may be thin film batteries used to provide energization. In some exemplary embodiments, these thin film batteries may comprise one or more of the layers that are stacked upon each other, in this case layers 730 may represent the battery layers, with multiple components in the layers.

As may be seen in nearly all of the layers, there may be interconnections that are made between two layers that are stacked upon each other. In the state of the art there may be numerous manners to make these interconnections; however, as demonstrated the interconnection may be made through solder ball interconnections between the layers. In some cases only these connections may be required, however in other cases the solder balls may contact other interconnection elements, as for example with a component having through layer vias.

In other layers of the stacked integrated component media insert, a layer dedicated to interconnection of various components in the interconnect layers may be found, as for example, layer 725. This layer may comprise vias and routing lines that pass signals from various components to others. For example, layer 725 may provide the various battery elements connections to a power management unit 720, which includes supply 740 and battery charger 765, that may be present in the technology layer components of layer 715. As well, the interconnection layer 725 may make connections between components in the technology layer and components outside the technology layer; as may exist for example in the integrated passive device component 760 shown as item 755. There may be numerous manners for routing of electrical signals that may be supported by the presence of dedicated interconnect layers.

There may be numerous layers identified as technology layers in a given application; however, in this exemplary embodiment there is a single layer 715. These features represent a diversity of technology options that may be included in media inserts. In some exemplary embodiments, the layer may include CMOS, BiCMOS, Bipolar, or memory based technologies. Alternatively, the layer may include different technology families within a same overall family; as for example layer 715 may include electronic elements produced using a 0.5 micron CMOS technology and also may include elements produced using a 20 nanometer CMOS technology. It may be apparent that many other combinations of various electronic technology types would be consistent within the art described herein.

In some exemplary embodiments, the media insert may include locations for electrical interconnections to components outside the media insert. In other exemplary embodiments; however, the media insert may also include interconnection to external components in a wireless manner. In such cases, the use of antennas may provide exemplary manners of wireless communication. In some such exemplary embodiments, a layer may exist, as shown as item 735, where such an exemplary antenna may be supported in the layer. In many cases, such an antenna layer may be located on the top or bottom of the stacked integrated component device within the media insert.

In some of the exemplary embodiments discussed herein, the battery elements may be included as elements in at least one of the stacked layers themselves. It may be noted as well that other exemplary embodiments may be possible where the battery elements are located externally to the stacked integrated component layers. Still further diversity in exemplary embodiments may derive from the fact that a separate battery or other energization component may also exist within the media insert, or alternatively these separate energization components may also be located externally to the media insert.

At item 710, a fluorescence detection element may be attached to a stacked integrated component. The fluorescence detection component may be attached as a portion of a layer in some exemplary embodiments. In other exemplary embodiments, the entire fluorescence detection element may also comprise a similarly shaped component as the other stacked components. The various diversity of types of fluorescence based analysis elements that have been discussed herein may be consistent with a stacked integrated component device, where other features such as light sources and light sensors either are a portion of a layer or alternatively attached to a stacked integrated component.

### Control Systems for Ophthalmic Devices with Integrated Fluorescence based Analysis Components

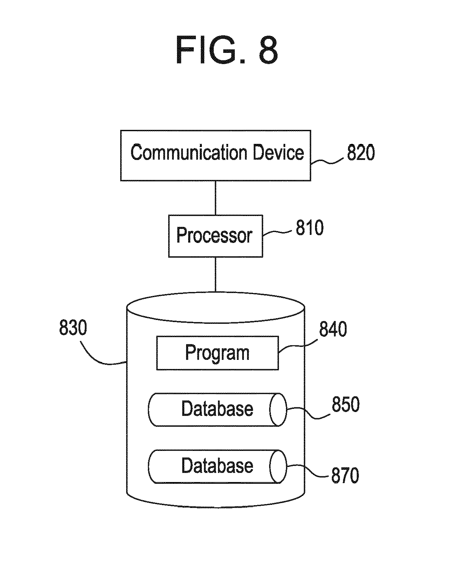

Referring now to Fig. 8 a controller is illustrated that may be used in accordance with some exemplary embodiments of the present invention. The controller includes one or more processors 810, which may include one or more processor components coupled to a communication device 820.

The processors 810 may be coupled to a communication device configured to communicate energy via a communication channel. The communication device may be used to communicate electronically with components within the ophthalmic insert within the ophthalmic device. The communication device 820 may also be used to communicate, for example, with one or more controller apparatus or manufacturing equipment components during the production of ophthalmic devices incorporating fluorescence based analysis elements.

The processor 810 may also be in communication with a storage device 830. The storage device 830 may comprise any appropriate information storage device, including combinations of magnetic storage devices (e.g., magnetic tape and hard disk drives), optical storage devices, and/or semiconductor memory devices such as Random Access Memory (RAM) devices and Read Only Memory (ROM) devices.

The storage device 830 may store a program 840 for controlling the processor 810. The processor 810 performs instructions of a software program 840, and thereby operates in accordance with the present invention. For example, the processor 810 may receive information descriptive of media insert placement, component placement, and the like. The storage device 830 may also store ophthalmic related data in one or more databases 850 and 870. The database may include customized specific control sequences for controlling the function of a fluorescence-based analytical system. The database may also include parameters and controlling algorithms for the control of fluorescence-based analysis components that may reside in the ophthalmic device as well as data that result from their action. In some exemplary embodiments, that data may be ultimately communicated to a reception device externally located to the ophthalmic device.

It is important to note that all of the components described herein may be sized and configured for use in ophthalmic devices or applications. In addition, the components are preferably biocompatible or encapsulated in biocompatible materials.

Although shown and described in what is believed to be the most practical and preferred embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope of the appended claims.

## Claims

1. An Ophthalmic Lens media insert device comprising:
a front annular insert piece and a rear annular insert piece, wherein the front annular insert piece is sealed to the rear annular insert piece forming an annular media insert comprising a sealed component cavity within;
an energy source within the sealed component cavity;
at least a first interconnect at least partially located within the sealed component cavity;
an electronic circuit within the sealed component cavity; and
a tab protruding from the annular insert piece, the tab configured to support a fluorescence analysis component and at least one electrical interconnect that connects the fluorescence analysis component to the electronic circuit.

2. The ophthalmic lens media insert according to claim 1, wherein the tab is a formed portion of the front annular insert piece, and
wherein the fluorescence analysis component is supported upon the tab portion of the rear annular insert piece.

3. The ophthalmic lens media insert according to claim 1 wherein the tab is a formed portion of the rear annular insert piece, and
wherein the fluorescence analysis component is supported upon the tab portion of the rear annular insert piece.

4. The ophthalmic lens media insert according to claim 1 wherein:
the tab is a formed portion of both the front annular insert piece and the rear annular insert piece and wherein the fluorescence analysis component is supported within a cavity of the tab formed by the tab region of the front annular insert piece and the rear annular insert piece.

5. An ophthalmic contact lens device comprising:
a front annular insert piece and a rear annular insert piece, wherein the front annular insert piece is sealed to the rear annular insert piece forming an annular media insert comprising a sealed component cavity within;
an energy source within the sealed component cavity;
at least a first interconnect at least partially located within the sealed component cavity;
an electronic circuit within the sealed component cavity;
a tab protruding from the annular insert piece, the tab configured to support a fluorescence analysis component and at least one electrical interconnect that connects the fluorescence analysis component to the electronic circuit; and
a hydrogel skirt containing the media insert.

6. The ophthalmic contact lens device according to claim 5 further comprising:
a stabilization feature, wherein the stabilization feature aids in locating the fluorescence analysis component in space when the ophthalmic contact lens device is worn by a user.

7. The ophthalmic contact lens device according to claim 5, wherein
the electronic circuit comprises a stacked integrated component device.

8. An ocular fluid analysis system for an ophthalmic device comprising:
an energized ophthalmic device comprising an energy source forming part of the ophthalmic device, wherein the energized ophthalmic device is suitable to be worn while placed in contact with the ocular fluid of a user's eye;
a fluorescence analytical system in electrical communication with the energy source, wherein the fluorescence analytic system is configured to measure a fluorescence signal in the ocular environment;
a fluorescence analysis probe that is embedded within ocular tissue, wherein the fluorescence analysis probe interacts with at least a first compound in the fluid of its location and emits a characteristic signal based at least in part upon the concentration of the first compound in interacts with and an excitation signal it receives from the fluorescence analytical system; and
a processor forming part of the ophthalmic device capable of executing a program including storing values related to the fluorescence signal observed by the fluorescence analysis system, wherein the program is configured to output a signal when a transmission command is received from an external device, wherein the output signal encodes some of the stored values into the output signal.

9. An ocular fluid analysis system for an ophthalmic device comprising:
an energized ophthalmic device comprising an energy source forming part of the ophthalmic device, wherein the energized ophthalmic device is suitable to be worn while placed in contact with the ocular fluid of a user's eye;
a fluorescence analytical system in electrical communication with the energy source, wherein the fluorescence analytic system is configured to measure a fluorescence signal in the ocular environment;
a fluorescence analysis probe that is embedded within an ocular tissue, wherein the fluorescence analysis probe interacts with at least a first compound in the fluid of its location and emits a characteristic signal based at least in part upon the concentration of the first compound in interacts with and an excitation signal it receives from the fluorescence analytical system; and
a processor forming part of the ophthalmic device capable of executing a program including evaluating a detected fluorescence signal against a preprogrammed threshold value for one or more ocular fluid properties indicated by the fluorescence signal characteristics, wherein the said program is configured to output a signal when the measurements are outside the corresponding preprogrammed threshold values.

10. A method of monitoring an ophthalmic fluid comprising:
providing an ophthalmic contact lens comprising a fluorescence analytical system to a user; and
receiving a data value that relates to a transmission originating from the ophthalmic contact lens.
